# EUROPEAN PATENT SPECIFICATION

(11) **EP 2 814 643 B1**
(45) Date of publication and mention of the grant of the patent: **24.03.2021**
(21) Application number: 13749878.8
(22) Date of filing: 15.02.2013
(51) Int. Cl.: B25J 13/00, B25J 19/02, A61F 2/60, A61F 2/68, A61F 2/72, A61F 2/70, A61F 2/76

(54) **CONTROL SYSTEMS AND METHODS FOR GAIT DEVICES**
STEUERUNGSSYSTEME UND -VERFAHREN FÜR GANGVORRICHTUNGEN
SYSTÈMES ET PROCÉDÉS DE COMMANDE POUR DES DISPOSITIFS DE MARCHE

(30) Priority: 17.02.2012 US 201261600141 P
(43) Date of publication of application: 24.12.2014
(73) Proprietor: Össur Iceland ehf, 110 Reykjavik (IS)
(72) Inventor: HOLGATE, Matthew Aaron, Chandler, Arizona 85286 (US)
(74) Representative: KIPA AB
(86) International application number: PCT/US2013/026285
(87) International publication number: WO 2013/123291

(56) References cited:
- WO-A2-2006/076164
- WO-A2-2011/005482
- WO-A2-2011/096965
- US-A1- 2005 070 834
- US-A1- 2010 161 077
- US-B2- 7 811 333
- US-B2- 7 811 333
- US-B2- 8 057 550

## Description

### BACKGROUND OF THE INVENTION

### 1. Field of the Invention

The field of the present invention is a gait device and methods of controlling gait devices, and particularly methods for prosthetic, orthotic, and robotic gait devices.

### 2. Background

Many control systems and methods have been designed for prosthetic, orthotic, and robotic gait devices. Nonetheless, there is still a need for gait devices and methods of controlling such gait devices that processes user signals quickly and accurately, while providing smooth and continuous control of the gait devices.

WO 2011/005482 A2 discloses a controllable transverse rotation adaptor to allow lower limb amputees to adjust torsional stiffness. A method is disclosed for dynamically enhancing performance of a prosthesis by enabling control of a rotational characteristic of a prosthesis during a transverse plain rotation. A desired torque is input to the control system and the difference between this desired torque and an actual load torque is determined by a summing section, yielding a torque error. The torque error is then passed to a PID controller that produces a motor torque input that is applied to transfer functions relating the load torque and a load angle. The load angle is provided as an input. The transfer functions include the gear ratio, gear box efficiency and polar moment of inertia, as well as a constant.

WO 2006/076164 A2 discloses a biological interface apparatus for controlling a joint movement device. The biological interface apparatus collects multicellular signals emanating from one or more living cells of a patient and transmits processed signals to the joint movement device. The interface apparatus includes a sensor for detecting multicellular signals and a plurality of electrodes. The joint movement device applies a force to one or more joints such as a patient joint or a joint of a prosthetic device.

US 7 811 333 B2 discloses systems and methods for processing limb motion and particularly a control system for processing a time series of signals associated with the movement of a device associated with a limb.

### SUMMARY

It is therefore the object of the present invention to provide an improved method of controlling a gait device and a corresponding gait device.

This object is solved by the subject matter of the independent claims.

Preferred embodiments are defined by the dependent claims.

The invention is directed to a gait device and methods of controlling gait devices. In one aspect of the invention, a method for controlling gait devices includes the steps of measuring kinematic and/or loading states of limb or robotic segments; conditioning the resulting state measurement by any combination or order of integration, differentiation, filtering, and amplification; transforming the conditioned state measurement by coordinate transformation; optionally conditioning the transformed state measurements a second time in a manner similar to the first conditioning step; and using the transformed or conditioned transformed state measurements as independent variables in a predetermined reference function to calculate a desired reference command for any number of actuators.

### BRIEF DESCRIPTION OF THE DRAWINGS

In the drawings, wherein like reference numerals refer to similar components:
FIG. 1 is a schematic representation of a method for controlling gait devices;
FIG. 2 is a perspective view of a gait augmentation robot, showing an example of coordinate systems of kinematic states;
FIG. 3 is a schematic representation of a second method for controlling gait devices;
FIG. 4A is a front view of a representation of an amputee having a prosthesis;
FIG. 4B is a perspective view of the amputee shown in FIG. 4A, showing a coordinate system used for controlling gait devices; and
FIG. 5 is a schematic representation of a control system, implementing a method for controlling gait devices.

### DETAILED DESCRIPTION

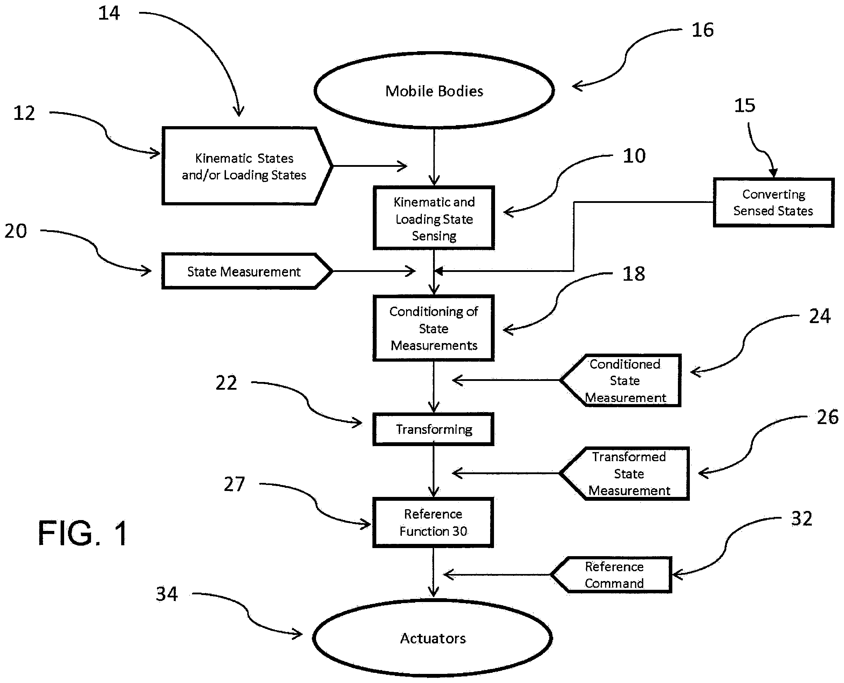

Referring to FIG. 1, a method for controlling gait devices includes: sensing **10** kinematic states **12** and/or loading states **14** generated by mobile bodies **16,** converting **15** sensed states into state measurements **20,** conditioning **18** state measurements **20** to yield conditioned state measurements **24,** transforming **22** the conditioned state measurements **24** into transformed state measurements **26,** and inputting **27** the transformed state measurements **26** into a reference function **30** to derive a reference command **32.** The reference command **32** is then used by one or more actuators **34** to aid in control of one or more gait devices (not shown).

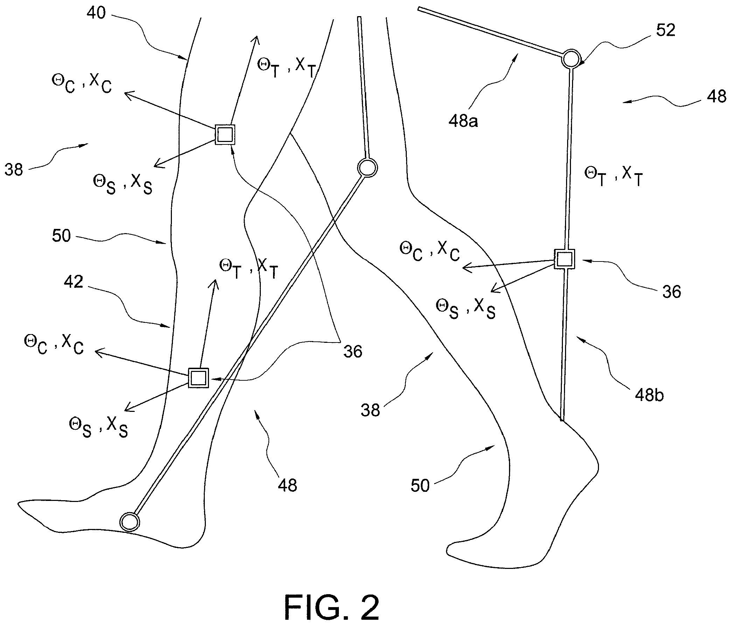

As used herein, the term "mobile body" is defined as a limb segment or robotic segment. As used herein, the term "kinematic state" used in connection with a mobile body, is defined as an angular position, linear position, linear velocity, angular velocity, linear acceleration, or angular acceleration associated with a mobile body with reference to a fixed world frame or a frame fixed to any other mobile body. Referring to FIG. 2, the kinematic state **12** can be measured using any type of sensor(s) **36** or sensor system affixed to limb segments **38,** such as thigh segments **40** or shank segments **42** of human legs, for example. Sensors **36** can also be affixed to robotic segments **48,** which may include multiple segments. FIG. 2 shows robotic segments **48** having upper segments **48a** and lower segments **48b.**

The sensors **36** are configured to measure velocities, accelerations, angular positions and/or linear positions in coordinate frames, which are oriented with the limb segment or robotic segment to which they are affixed. These coordinate frames have three orthogonal axes: (1) the sagittal axis (Θ_{S}, X_{S}), (2) the coronal axis (Θ_{C}, X_{C}), and (3) the transverse axis (Θ_{T}, X_{T}). The sagittal axis (Θ_{S}, X_{S}) is oriented normal to the sagittal plane of the segment, while the coronal axis (Θ_{C}, X_{C}) is oriented normal to the coronal plane of the segment and the transverse axis (Θ_{T}, X_{T}) is oriented normal to the transverse plane of the segment. As such, each sensor **36** is oriented so that its axis of measurement is any linear combination of three unit vectors in the direction of the sagittal, coronal, and transverse axes.

As used herein, the term "loading state" used in connection with a mobile body, is defined as a moment or force experienced by a mobile body. Also referring to FIG. 2, a moment or force can be measured, using any type of sensor **36** or sensor system. The sensors **36** can be located on one or more limb segments **38,** robotic segments **48,** limb joint **50,** robotic joint **52,** or any type of limb-robot interface.

With regard to the loading state **14,** the sensors **36** measure force or moment experienced at the point in the limb or robot in coordinate frames, where the coordinate frames are defined by the sagittal, coronal, and transverse axes. Each sensor **36** is also oriented so that its axis of measurement is any linear combination of the three unit vectors in the direction of the sagittal, coronal, and transverse axes.

Referring back to FIG. 1, after sensing of the kinematic states **12** and/or loading states **14** by sensors **36** or sensor system, converting **15** of the sensed states occurs. In this step, the sensed states are converted from an output of the sensor **36** to a desired unit of measurement that yields a state measurement **20.**

State measurements **20** are then conditioned to yield conditioned state measurements **24.** Conditioning **18** is realized by any filtering method, including, but not limited to Kalman filtering, transfer function use, integration, differentiation, and amplification. These filtering methods may be performed as many times as desired.

Amplification may result from a gain of any nonzero number, including by a unity gain. In addition, conditioning may also be realized by any combination and order of filtering, integration, differentiation, and/or amplification. Filtering can be employed for multiple uses, including but not limited to: reduction of noise in state measurements, reduction of inaccuracies in state measurements, or alteration of state measurements. For example, alteration of state measurements may be performed in a manner similar to integration or differentiation such that drift in numerical integration or noise in numerical differentiation is eliminated.

Transforming **22** of conditioned state measurements **24** is generally described as changing coordinate systems to yield transformed state measurements **26,** which are realized by isometric or non-isometric transformations. These types of transformations include rotations and dilations. Other types of transformations, however, include identity transformations, projections, changes to other coordinate systems, and changes of scale. The projections may either be orthogonal or oblique. In addition, other coordinate systems may include polar coordinate systems, barycentric coordinate systems, and other similar types of coordinate systems. Changes of scale may be log scale or any other function of scale. Moreover, these transformations may include any transformation where the transformed state measurements are any mathematical function of the conditioned state measurements; or any combination in any order of transformations, projections, changes of coordinate system, changes of scale, mathematical functions, etc.

A transformed state measurement coordinate system is not restricted to have the same number of dimensions as the conditioned state measurement coordinate system. In fact, there may be more or less transformed state measurements than conditioned state measurements. Transformation is generally employed so that a robust relationship between the conditioned state measurements and the desired output reference command can be found. However, transformation is not limited to this use.

Transformed state measurements **26** are used as arguments to one or more reference functions **30.** The transformed state measurements **26** are therefore used to calculate reference commands **32,** using the reference functions **30.** Each reference function **30** is a function that relates the transformed state measurements **26** as independent variables to the reference command as a dependent variable.

The reference function **30** can be represented in any way that accepts inputs and that outputs a unique value for each combination of inputs. As such, the reference function may be represented using any suitable method. Suitable methods of representation include look up tables, mathematical functions, or combinations of tables and mathematical functions.

The reference function **30** is determined by recording data from sensors **36** and then by using the aforementioned method(s) to obtain the transformed state measurements **26** combined with either a recording or calculation of a desired reference command. The reference function **30** is also made to match data from one or more gait activities. Such activities include as walking, running, traversing slopes or stairs, avoiding obstacles, and other similar activities.

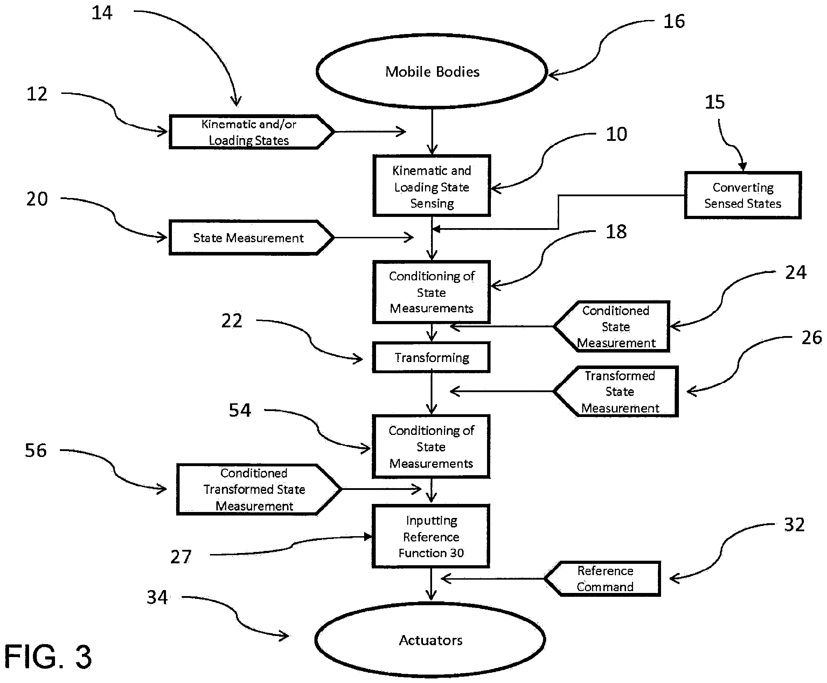

As shown schematically in FIG. 3, after transforming **22** of the conditioned state measurements **24,** one or more of the transformed state measurements **26** may be conditioned in an additional conditioning step **54.** This step occurs before the transformed state measurements are used as arguments for the reference function **30.** In this conditioning step **54** conditioned transformed state measurements **56** result.

Here, conditioning may also be realized by any filtering method. Such filtering methods include, but are not limited to Kalman filtering, transfer function use, integration, differentiation, and amplification. Integration and differentiation may be performed as many times as is desired; while amplification may result from a gain of any nonzero number, not including a unity gain.

Filtering method(s) may include filtering, integration, differentiation, and/or amplification performed in any combination and in any order. Any transformed state measurements and conditioned transformed state measurements are used as arguments to one or more previously determined reference command functions. These measurements are then used to calculate the desired reference commands.

Each reference command function is a function that relates the transformed state measurements and the conditioned transformed state measurements as independent variables to the desired reference command as a dependent variable. The reference command function is made to match data from any combination of two or more gait activities such as walking, running, traversing slopes or stairs, obstacle avoidance, or similar activities.

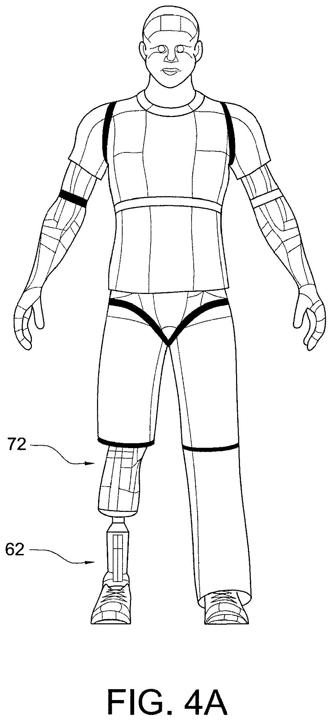

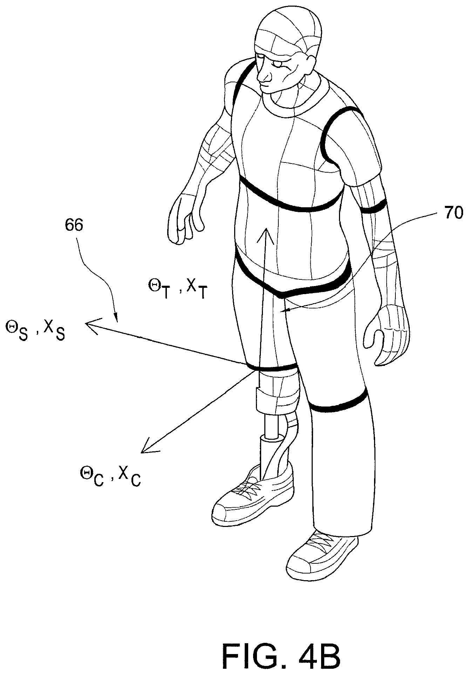

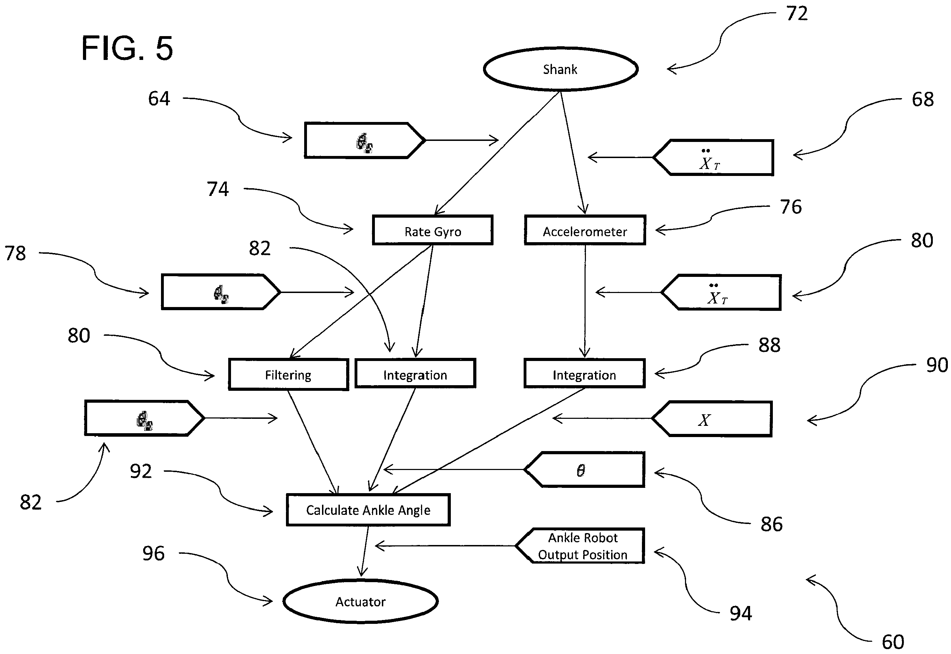

Referring to FIGs. 4A, 4B, and 5, an implementation of the method is shown as a control system **60** for an ankle prosthesis **62.** In the control system **60,** an angular velocity kinematic state **64** in the sagittal direction **66** and an acceleration kinematic state **68** in the transverse direction **70** of a shank **72** are measured. In this implementation, measurements are taken using a rate gyro **74** and an accelerometer **76,** respectively, to yield an angular velocity state measurement **78** and an acceleration state measurement **79.**

The angular velocity state measurement **78** is conditioned by filtering 80 to yield an angular velocity conditioned state measurement **82,** while the angular velocity state measurement **78** is conditioned by integration **84** to get an angle conditioned state measurement **86,** and the acceleration state measurement **79** is conditioned by double integration **88** to yield a position conditioned state **90.** The angular velocity conditioned state measurement **82,** angle conditioned state measurement **86,** and position conditioned state measurement **90** are each transformed by identity transformation (not shown) resulting in no change to the conditioned state measurements **82, 86, 90.** The conditioned state measurements **82, 86, 90** are then used as arguments in the ankle angle reference command function **92** which yields an ankle robot output position reference command **94.** The command function **94** is then used by the actuator **96** of the ankle prosthesis **62.**

The control systems and methods for gait devices described herein have several benefits. For example, the continuous nature of the reference command calculation is beneficial because the method continuously measures a limb or robot segment directly and computes a reference command from a continuous differentiable function. As a result, the reference command is less likely to make sudden jumps or undesirable oscillations. Moreover, because the reference command is a function of measured quantities, generally there is no decision making and no state machine switching of states. Dealing with decision making and state transitions is known to be error prone, often resulting in undesirable operation when a state is chosen incorrectly.

The aforementioned control systems and methods may be employed in a wide field of applications. Some examples, which are in no way exhaustive, include controlling lower limb prostheses and orthotic devices and assisting in the operation of exoskeleton devices. Also, the method may be employed in computer animation, gaming, and other fields where the control of robotic and bionic machines benefit from characterization of cyclic patterns.

Thus, gait devices and methods for controlling gait devices are disclosed, wherein the scope of the invention is defined by the appended claims.

## Claims

1. A method of controlling a gait device, comprising: providing a sensor (36); measuring kinematic and/or loading states of a mobile body (16) using the sensor to obtain a state measurement (20), wherein the mobile body includes a limb segment or a robot segment; conditioning the state measurement to obtain a conditioned state measurement (24), wherein the conditioning is realized by filtering, integration, differentiation, amplification and/or any combination thereof;
transforming the conditioned state measurement by a coordinate transformation to obtain a transformed state measurement (26);
providing a predetermined reference function (30) that relates the transformed state measurement as an independent variable to a reference command (32) as a dependent variable, wherein the reference command (32) is for use by one or more actuators (34; 96) to control of one or more gait devices, wherein the reference function matches data from a plurality of gait activities and the output reference command of the reference function is unique for each input of the reference function; and
calculating the reference command (32) by inputting the transformed state measurement into the reference function.

2. The method of claim 1, wherein measuring the kinematic state further includes measuring an angular position, linear position, linear velocity, angular velocity, linear acceleration, or angular acceleration.

3. The method of claim 1, wherein measuring the loading state further includes measuring a moment or force applied to the mobile body.

4. The method of claim 1, wherein conditioning the state measurement (20) is realized by a conditioning method selected from the group consisting of Kalman filtering, use of a transfer function, integration, differentiation, amplification by a non-zero gain, amplification by a unity gain, and addition of a constant offset.

5. The method of claim 1, wherein transforming the conditioned state measurement is realized by a transformation method selected from the group consisting of rotations, dilations, orthogonal or oblique projections, identity transformation, changes of coordinate systems, changes of scale, and mathematical functions.

6. A method of controlling a gait device, comprising:
providing a sensor (36);
measuring kinematic and/or loading states of a mobile body using the sensor to obtain a state measurement (20);
transforming the state measurement by a coordinate transformation to obtain a transformed state measurement;
conditioning the transformed state measurement to obtain a conditioned transformed state measurement, wherein the conditioning is realized by filtering, integration, differentiation, amplification and/or any combination thereof;
providing a predetermined reference function (30) that relates the transformed state measurement as an independent variable to a reference command (32) as a dependent variable, wherein the reference command (32) is for use by one or more actuators (34; 96) to control of one or more gait devices, wherein the reference function (30) matches data from a plurality of gait activities and the output reference command (32) of the reference function (30) is unique for each input of the reference function (30);
generating the reference command (32) by inputting the conditioned transformed state measurement into the reference function.

7. The method of claim 6, wherein measuring the kinematic state further includes measuring an angular position, linear position, linear velocity, angular velocity, linear acceleration, or angular acceleration.

8. The method of claim 6, wherein measuring the loading state further includes measuring a moment or force applied to or internal to the mobile body.

9. The method of claim 6, wherein conditioning the state measurement (20) is realized by a conditioning method selected from the group consisting of Kalman filtering, use of a transfer function, integration, differentiation, amplification by a non-zero gain, amplification by a unity gain, and addition of a constant offset.

10. The method of claim 6, wherein transforming the conditioned state measurement is realized by a transformation method selected from the group consisting of rotations, dilations, orthogonal or oblique projections, identity transformation, changes of coordinate systems, changes of scale, and mathematical functions.

11. A gait device, comprising:
a prosthetic limb device including an actuator (34; 96);
a sensor coupled to the prosthetic limb device; and
a control system coupled to the prosthetic limb device, wherein the control system is configured to obtain a state measurement (20) from the sensor by measuring kinematic and/or
loading states and produce a reference command (32) based a plurality of gait activities to control the actuator using a predetermined reference function (30), wherein the reference function relates a transformed state measurement as an independent variable to a reference command (32) as a dependent variable, the state measurement (20) and/or the transformed state measurement being conditioned to obtain a conditioned state measurement (24) and/or a conditioned transformed state measurement, the conditioning realized by filtering, integration, differentiation, amplification and/or any combination thereof, wherein the reference command (32) is for use by the actuator (34; 96) to control the gait device, wherein .the reference function matches data from the plurality of gait activities and the output reference command of the reference function is unique for each input of the reference function.

12. The gait device of claim 11, wherein the prosthetic limb device is configured to be worn by a user to replace a limb or assist movement.

13. The gait device of claim 11, wherein the prosthetic limb device includes an ankle prosthesis.

14. The gait device of claim13, wherein the sensor includes a rate gyro or an accelerometer.

## Patentansprüche

1. Ein Verfahren zur Steuerung einer Gehvorrichtung mit:
Einsatz eines Sensors (36),
Messung kinematischer und/oder von Belastungszuständen eines mobilen Körpers (16), wobei der Sensor zur Zustandsmessung (20) dient und der mobile Körper ein Gliedmaßesegment oder ein Robotersegment umfasst,
Aufbereitung der Zustandsmessung zu einer aufbereiteten Zustandsmessung (24), wobei die Aufbereitung durch Filterung, Integration, Differenzierung, Verstärkung und/oder eine beliebige Kombination von diesen erfolgt,
Umrechnung der aufbereiteten Zustandsmessung über eine Koordinatentransformation zu einem umgerechneten Belastungszustand (26),
Bereitstellung einer vorher festgelegten Referenzfunktion (30), die die umgerechnete Zustandsmessung als unabhängige Variable in Bezug zu einem Referenzbefehl (32) als abhängige Variable setzt, wobei der Referenzbefehl (32) von einem oder mehreren Stellgliedern (34, 96) zur Steuerung einer oder mehrerer Gehvorrichtungen verwendet wird und die Referenzfunktion Daten einer Vielzahl von Gehaktivitäten abgleicht und der ausgegebene Referenzbefehl der Referenzfunktion für jeden Eingang der Referenzfunktion einzigartig ist, und
Berechnung des Referenzbefehls (32) durch Einsetzen der umgerechneten Zustandsmessung in die Referenzfunktion.

2. Das Verfahren gemäß Anspruch 1, wobei die Messung des kinematischen Zustands weiterhin die Messung von Winkelposition, linearer Position, linearer Geschwindigkeit, Winkelgeschwindigkeit, linearer Beschleunigung oder Winkelbeschleunigung umfasst.

3. Das Verfahren gemäß Anspruch 1, wobei die Messung des Belastungszustands weiterhin die Messung eines Drehmoments oder einer auf den Körper ausgeübten Kraft beinhaltet.

4. Das Verfahren gemäß Anspruch 1, wobei die Aufbereitung der Zustandsmessung (20) über eine Aufbereitungsmethode aus der folgenden Aufzählung erfolgt: Kalman-Filterung, Verwendung einer Transferfunktion, Integration, Differenzierung, Nicht-Null-Verstärkung, Eins-Verstärkung und Addieren eines konstanten Offsets.

5. Das Verfahren gemäß Anspruch 1, wobei die Umrechnung der aufbereiteten Zustandsmessung über ein Umrechnungsverfahren aus der folgenden Gruppe erfolgt: Drehung, Erweiterung, orthogonale oder schiefachsige Projektion, Identitätstransformation, Änderungen des Koordinatensystems, Maßstabsänderung und mathematische Funktionen.

6. Ein Verfahren zur Steuerung einer Gehvorrichtung mit:
Einsatz eines Sensors (36),
Messung kinematischer und/oder von Belastungszuständen eines mobilen Körpers (16), wobei der Sensor zur Zustandsmessung (20) dient
Umrechnung der Zustandsmessung über eine Koordinatentransformation zu einem umgerechneten Belastungszustand,
Aufbereitung der umgerechneten Zustandsmessung zu einer aufbereiteten, umgerechneten Zustandsmessung, wobei die Aufbereitung durch Filterung, Integration, Differenzierung, Verstärkung und/oder eine beliebige Kombination von diesen erfolgt,
Bereitstellung einer vorher festgelegten Referenzfunktion (30), die die umgerechnete Zustandsmessung als unabhängige Variable in Bezug zu einem Referenzbefehl (32) als abhängige Variable setzt, wobei der Referenzbefehl (32) von einem oder mehreren Stellgliedern (34, 96) zur Steuerung einer oder mehrerer Gehvorrichtungen verwendet wird und die Referenzfunktion (30) Daten einer Vielzahl von Gehaktivitäten abgleicht und der ausgegebene Referenzbefehl (32) der Referenzfunktion (30) für jeden Eingang der Referenzfunktion (30) einzigartig ist,
Generierung des Referenzbefehls (32) durch Einsetzen der aufbereiteten, umgerechneten Zustandsmessung in die Referenzfunktion.

7. Das Verfahren gemäß Anspruch 6, wobei die Messung des kinematischen Zustands weiterhin die Messung von Winkelposition, linearer Position, linearer Geschwindigkeit, Winkelgeschwindigkeit, linearer Beschleunigung oder Winkelbeschleunigung umfasst.

8. Das Verfahren gemäß Anspruch 6, wobei die Messung des Belastungszustands weiterhin die Messung eines Drehmoments oder einer auf den Körper ausgeübten oder ihm innewohnenden Kraft beinhaltet.

9. Das Verfahren gemäß Anspruch 6, wobei die Aufbereitung der Zustandsmessung (20) über eine Aufbereitungsmethode aus der folgenden Aufzählung erfolgt: Kalman-Filterung, Verwendung einer Transferfunktion, Integration, Differenzierung, Nicht-Null-Verstärkung, Eins-Verstärkung und Addieren eines konstanten Offsets.

10. Das Verfahren gemäß Anspruch 6, wobei die Umrechnung der aufbereiteten Zustandsmessung über ein Umrechnungsverfahren aus der folgenden Gruppe erfolgt: Drehung, Erweiterung, orthogonale oder schiefachsige Projektion, Identitätstransformation, Änderungen des Koordinatensystems, Maßstabsveränderung und mathematische Funktionen.

11. Eine Gehvorrichtung mit:
einer Gliedmaßenprothese, die ein Stellglied (34,96) umfasst,
einem an die Gliedmaßenprothese gekoppelten Sensor und
einem an die Gliedmaßenprothese gekoppelten Kontrollsystem, wobei das Kontrollsystem so ausgelegt ist, dass es eine Zustandsmessung (20) vom Sensor durch Messung kinematischer und/oder von Belastungszuständen erhält und einen Referenzbefehl (32) auf Grundlage einer Mehrzahl von Gehaktivitäten zur Kontrolle des Stellglieds unter Verwendung einer vorgegebenen Referenzfunktion (30) ausgibt, wobei die Referenzfunktion eine umgerechnete Zustandsmessung als unabhängige Variable in Bezug zu einem Referenzbefehl (32) als abhängige Variable setzt, die Zustandsmessung (20) und/oder die umgerechnete Zustandsrechnung aufbereitet werden und eine aufbereitete Zustandsmessung (24) und/oder eine aufbereitete, umgerechnete Zustandsmessung ergeben und die Aufbereitung durch Filterung, Integration, Differenzierung, Verstärkung und/oder eine beliebige Kombination von diesen erfolgt, wobei der Referenzbefehl (32) vom Stellglied (34, 96) zur Steuerung der Gehvorrichtung verwendet wird und die Referenzfunktion Daten der Vielzahl von Gehaktivitäten abgleicht und der ausgegebene Referenzbefehl der Referenzfunktion für jeden Eingang der Referenzfunktion einzigartig ist.

12. Die Gehvorrichtung gemäß Anspruch 11, wobei die Gliedmaßenprothese zum Tragen durch einen Anwender als Ersatz für eine Gliedmaße oder zur Unterstützung bei Bewegungen getragen wird.

13. Die Gehvorrichtung gemäß Anspruch 11, wobei die Gliedmaßenprothese eine Sprunggelenkprothese umfasst.

14. Die Gehvorrichtung gemäß Anspruch 13, wobei der Sensor einen Drehbewegungsmesser oder einen Beschleunigungsmesser umfasst.

## Revendications

1. Procédé de commande d'un dispositif de marche, comprenant les étapes consistant à :
fournir un capteur (36) ;
mesurer des états cinématique et/ou de charge d'un corps mobile (16) en utilisant le capteur pour obtenir une mesure d'état (20), où le corps mobile inclut un segment de membre ou un segment robotisé ;
conditionner la mesure d'état pour obtenir une mesure d'état conditionnée (24), où le conditionnement est réalisé par filtrage, intégration, calcul de dérivée, amplification et/ou toute combinaison de ceux-ci ;
transformer la mesure d'état conditionnée par une transformation de coordonnées pour obtenir une mesure d'état transformée (26) ;
fournir une fonction de référence prédéterminée (30) qui associe la mesure d'état transformée à titre de variable indépendante à un ordre de référence (32) à titre de variable dépendante, où l'ordre de référence (32) est destiné à être utilisé par un ou plusieurs actionneurs (34 ; 96) pour la commande d'un ou de plusieurs dispositifs de marche, où la fonction de référence met en correspondance des données émanant d'une pluralité d'activités de marche et l'ordre de référence de sortie de la fonction de référence est unique pour chaque entrée de la fonction de référence ; et
calculer l'ordre de référence (32) en entrant la mesure d'état transformée dans la fonction de référence.

2. Procédé selon la revendication 1, dans lequel le fait de mesurer l'état cinématique inclut en outre le fait de mesurer une position angulaire, une position linéaire, une vitesse linéaire, une vitesse angulaire, une accélération linéaire, ou une accélération angulaire.

3. Procédé selon la revendication 1, dans lequel le fait de mesurer l'état de charge inclut en outre le fait de mesurer un moment ou une force appliqué au corps mobile.

4. Procédé selon la revendication 1, dans lequel le fait de conditionner la mesure d'état (20) est réalisé par un procédé de conditionnement sélectionné parmi le groupe constitué d'un filtrage de Kalman, d'une utilisation d'une fonction de transfert, d'une intégration, d'un calcul de dérivée, d'une amplification par un gain non nul, d'une amplification par un gain unitaire, et d'un ajout d'un décalage constant.

5. Procédé selon la revendication 1, dans lequel le fait de transformer la mesure d'état conditionnée est réalisé par un procédé de transformation sélectionné parmi le groupe constitué de rotations, de dilatations, de projections orthogonales ou obliques, de transformation d'identité, de changements de systèmes de coordonnées, de changements d'échelle, et de fonctions mathématiques.

6. Procédé de commande d'un dispositif de marche, comprenant les étapes consistant à :
fournir un capteur (36) ;
mesurer des états cinématique et/ou de charge d'un corps mobile en utilisant le capteur pour obtenir une mesure d'état (20) ;
transformer la mesure d'état par une transformation de coordonnées pour obtenir une mesure d'état transformée ;
conditionner la mesure d'état transformée pour obtenir une mesure d'état transformée conditionnée, où le conditionnement est réalisé par filtrage, intégration, calcul de dérivée, amplification et/ou toute combinaison de ceux-ci ;
fournir une fonction de référence prédéterminée (30) qui associe la mesure d'état transformée à titre de variable indépendante à un ordre de référence (32) à titre de variable dépendante, où l'ordre de référence (32) est destiné à être utilisé par un ou plusieurs actionneurs (34 ; 96) pour la commande d'un ou de plusieurs dispositifs de marche, où la fonction de référence (30) met en correspondance des données émanant d'une pluralité d'activités de marche et l'ordre de référence de sortie (32) de la fonction de référence (30) est unique pour chaque entrée de la fonction de référence (30) ;
générer l'ordre de référence (32) en entrant la mesure d'état transformée conditionnée dans la fonction de référence.

7. Procédé selon la revendication 6, dans lequel le fait de mesurer l'état cinématique inclut en outre le fait de mesurer une position angulaire, une position linéaire, une vitesse linéaire, une vitesse angulaire, une accélération linéaire, ou une accélération angulaire.

8. Procédé selon la revendication 6, dans lequel le fait de mesurer l'état de charge inclut en outre le fait de mesurer un moment ou une force appliqué au ou interne au corps mobile.

9. Procédé selon la revendication 6, dans lequel le fait de conditionner la mesure d'état (20) est réalisé par un procédé de conditionnement sélectionné parmi le groupe constitué d'un filtrage de Kalman, d'une utilisation d'une fonction de transfert, d'une intégration, d'un calcul de dérivée, d'une amplification par un gain non nul, d'une amplification par un gain unitaire, et d'un ajout d'un décalage constant.

10. Procédé selon la revendication 6, dans lequel le fait de transformer la mesure d'état conditionnée est réalisé par un procédé de transformation sélectionné parmi le groupe constitué de rotations, de dilatations, de projections orthogonales ou obliques, de transformation d'identité, de changements de systèmes de coordonnées, de changements d'échelle, et de fonctions mathématiques.

11. Dispositif de marche, comprenant :
un dispositif de membre prothétique incluant un actionneur (34 ; 96) ;
un capteur couplé au dispositif de membre prothétique ; et
un système de commande couplé au dispositif de membre prothétique, où le système de commande est configuré pour obtenir une mesure d'état (20) à partir du capteur en mesurant des états cinématique et/ou de charge et produire un ordre de référence (32) sur la base d'une pluralité d'activités de marche pour commander l'actionneur en utilisant une fonction de référence prédéterminée (30), où la fonction de référence associe une mesure d'état transformée à titre de variable indépendante à un ordre de référence (32) à titre de variable dépendante, la mesure d'état (20) et/ou la mesure d'état transformée étant conditionnées pour obtenir une mesure d'état conditionnée (24) et/ou une mesure d'état transformée conditionnée, le conditionnement étant réalisé par filtrage, intégration, calcul de dérivée, amplification et/ou toute combinaison de ceux-ci, où l'ordre de référence (32) est destiné à être utilisé par l'actionneur (34 ; 96) pour commander le dispositif de marche, où la fonction de référence met en correspondance des données émanant de la pluralité d'activités de marche et l'ordre de référence de sortie de la fonction de référence est unique pour chaque entrée de la fonction de référence.

12. Dispositif de marche selon la revendication 11, dans lequel le dispositif de membre prothétique est configuré pour être porté par un utilisateur pour remplacer un membre ou assister le mouvement.

13. Dispositif de marche selon la revendication 11, dans lequel le dispositif de membre prothétique inclut une prothèse de cheville.

14. Dispositif de marche selon la revendication 13, dans lequel le capteur inclut un gyromètre ou un accéléromètre.
